(19) Europäisches Patentamt
European Patent Office
Office européen des brevets

(11) Numéro de publication: **0 239 826**
**B1**

# (12) FASCICULE DE BREVET EUROPEEN

(45) Date de publication du fascicule du brevet: **23.01.91**

(51) Int. Cl.$^5$: **A 61 K 7/42**

(21) Numéro de dépôt: **87103101.9**

(22) Date de dépôt: **05.03.87**

## (54) Compositions pour la coloration de la peau à base de dérivés d'indole.

(30) Priorité: **06.03.86 LU 86347**

(43) Date de publication de la demande: **07.10.87 Bulletin 87/41**

(45) Mention de la délivrance du brevet: **23.01.91 Bulletin 91/04**

(84) Etats contractants désignés: **AT BE CH DE FR GB IT LI NL SE**

(56) Documents cités:
**FR-A-2 321 875**
**FR-A-2 390 158**
**US-A-3 467 670**
**US-A-4 013 404**
**US-A-4 515 773**
**US-A-4 522 808**

**CHEMICAL ABSTRACTS, vol. 75, 1971, page 209, résumé no. 118189v, Columbus, Ohio, US; J.R. MERCHANT et al.: "Heterocyclic compounds. XII. Color tests, spectra, and reactions of some indole derivatives", & J. INDIAN. CHEM. SOC. 1971, 48(7), 613-19**

(73) Titulaire: **L'OREAL**
**14, Rue Royale**
**F-75008 Paris (FR)**

(72) Inventeur: **Lang, Gérard**
**44, Avenue Lacour**
**F-95210 Saint Gratien (FR)**
Inventeur: **Richard, Hervé**
**48, Rue de l'Ermitage**
**F-75020 Paris (FR)**
Inventeur: **Leduc, Madeleine**
**29 Rue des Boulets**
**F-75011 Paris (FR)**
Inventeur: **Junino, Alex**
**16, Rue Docteur Begonié**
**F-93180 Livry-Gargan (FR)**

(74) Mandataire: **Casalonga, Axel et al**
**BUREAU D.A. CASALONGA - JOSSE**
**Morassistrasse 8**
**D-8000 München 5 (DE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 239 826 B1

**Description**

La présente invention est relative à de nouvelles compositions cosmétiques destinées à conférer à la peau une coloration sensiblement analogue à la pigmentation résultant du bronzage naturel.

Le bronzage naturel est dû à la mélanisation résultant de l'exposition de l'épiderme humain à l'irradiation lumineuse ayant une longueur d'onde comprise entre 280 et 400 nanomètres.

On a déjà proposé dans le passé des compositions destinées à conférer à la peau, un bronzage artificiel et on peut citer en particulier des compositions à base de dihydroxyacétone. Le bronzage obtenu par l'intermédiaire de dihydroxyacétone résulte d'une réaction entre le produit actif et les composants protéiniques de la peau indépendamment d'une exposition au soleil.

L'application de tels composés présente cependant un inconvénient résultant du mécanisme à l'origine de l'apparition de la coloration et on obtient très souvent après les applications successives, des nuances différentes suivant que la peau présente ou non des parties cornées. La coloration obtenue s'élimine par ailleurs irrégulièrement au lavage ce qui conduit à des différences de nuances. Par ailleurs, les mélanoïdines développées ne protègent pas contre le rayonnement solaire.

La demanderesse a découvert que des dérivés de l'indole permettaient de développer une coloration de la peau proche de la pigmentation résultant d'un bronzage naturel. Cette coloration est due à leur transformation, avec ou sand activation du rayonnement solaire, en un pigment proche du pigment naturel à l'origine du bronzage. Le rayonnement ultraviolet permet en particulier d'activer l'apparition de cette coloration.

Les dérivés d'indole utilisables conformément à l'invention, sont pour partie connus en eux-mêmes. Les brevets US—A—3 154 734 et FR—A—2 390 158 décrivent les 5,6-dihydroxy indole substitués éventuellement en position 1, 2 et 3 par des groupements méthyle pour leur application à la teinture des cheveux.

Un objet de l'invention est constitué par l'utilisation de dérivés d'indole pour la coloration de la peau.

Un objet de l'invention est constitué également par de nouvelles compositions pour la coloration de la peau à base de dérivés d'indole.

Un autre objet de l'invention est constitué par le procédé de coloration de la peau mettant en oeuvre ces composés.

D'autres objets de l'invention apparaîtront à la lecture de la description et des exemples qui suivent.

Les composés destinées à être utilisés pour la coloration de la peau conformément à l'invention répondent de formule:

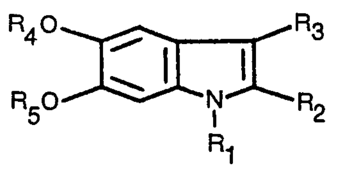

(I)

dans laquelle $R_1$ représente un atome d'hydrogène, un groupement alkyle inférieur ou un reste $-SiR_9R_{10}R_{11}$, $R_2$ et $R_3$ identiques ou différents, représentent un atome d'hydrogène, un groupement alkyle inférieur, un groupement carboxyle, un groupement alcoxycarbonyle inférieur ou un groupement $-COOSiR_9R_{10}R_{11}$; $R_4$ et $R_5$, identiques ou différents, représentent un atome d'hydrogène, un groupement alkyle linéaire ou ramifié en $C_1$—$C_{20}$, un groupement formyle, un groupement acyle linéaire ou ramifiée en $C_2$—$C_{20}$, un groupement alcénoyle linéaire ou ramifié en $C_3$—$C_{20}$, un groupement $R_6OSO_2-$, un groupement $-SiR_9R_{10}R_{11}$, un groupement $-P(O)(OR_6)_2$, un groupement aralkyle, ou bien $R_4$ et $R_5$, avec les atomes d'oxygène auxquels ils sont liés, forment un cycle contenant éventuellement un groupement carbonyle quand l'un au moins des groupements $R_1$, $R_2$ ou $R_3$ est différent d'hydrogène ou bien un groupement thiocarbonyle ou un reste $>P(O)OR_6$, un groupement $>CR_7R_8$ ou un groupement méthylène, $R_6$ représentant un atome d'hydrogène ou un groupement alkyle inférieur, $R_7$ représentant un atome d'hydrogène ou un groupement alkyle inférieur, $R_8$ représentant un groupement alcoxy inférieur ou un groupement mono ou dialkylamino et $R_9$, $R_{10}$ ou $R_{11}$, identiques ou différents, représentant des groupements alkyle inférieurs, linéaires ou ramifiés, l'un au moins des substituants $R_1$, $R_2$, $R_3$, $R_4$, $R_5$ étant différent de l'hydrogène; et les sels correspondants avec les métaux alcalins, alcalino-terreux ou d'amines.

Dans les groupements définis ci-dessus un groupement alkyle inférieur ou alcoxy inférieur désigne un groupement ayant 1 à 6 atomes de carbone.

Parmi les composés de formule (I), on peut citer les composés suivants figurant au tableau I.

TABLEAU I

| No. | Désignation du composé | Pt de fusion et d'ébullition (E) °C |
|---|---|---|
| 1 | Dibenzyloxy-5,6 indole | 114° |
| 2 | Benzyloxy-5 méthoxy-6 indole | 95—6° |
| 3 | Benzyloxy-6 méthoxy-5 indole | 148° |
| 4 | Hydroxy-6 méthoxy-5 indole | 111° |
| 5 | Hydroxy-5 méthoxy-6 indole | 116° |
| 6 | Diacétoxy-5,6 indole | 140° |
| 7 | Acétoxy-6 méthoxy-5 indole | 140° |
| 8 | Acétoxy-(5 ou 6)hydroxy-(6 ou 5)indole | 184° |
| 9 | Dibenzyloxy-5,6 carbéthoxy-2 indole | 140° |
| 10 | Dibenzyloxy-5,6 carboxy-2 indole | 201—2° |
| 11 | Dihydroxy-5,6 carbéthoxy-2 indole | 194° |
| 12 | Dihydroxy-5,6 carboxy-2 indole | 240°(d) |
| 13 | Dibenzyloxy-5,6 méthyl-2 indole | 116° |
| 14 | Dihydroxy-5,6 méthyl-2 indole | 216—8° |
| 15 | Dibenzyloxy-5,6 méthyl-3 indole | 114° |
| 16 | Dihydroxy-5,6 méthyl-3 indole | 156° |
| 17 | Formyloxy-(5 ou 6)hydroxy-(6 ou 5)indole | 161° |
| 18 | Acétoxy-(5 ou 6)formyloxy-(6 ou 5)indole | — |
| 19 | Formyloxy-6 méthoxy-5 indole | 118° |
| 20 | Benzyloxy-6 butoxy-5 indole | 116° |
| 21 | Butoxy-5 hydroxy-6 indole | 91° |
| 22 | Benzyloxy-5 butoxy-6 indole | — |
| 23 | Butoxy-6 hydroxy-5 indole | 105° |
| 24 | Hydroxy-(5 ou 6)triméthylsilyloxy-(6 ou 5)indole | 97° |
| 25 | Di(triméthylsilyloxy)-5,6 indole | 67°<br>* E 151° |
| 26 | [(Ethoxy-1 éthyl)-1,1 dioxy]-5,6 indole | 69°<br>* E 160° |
| 27 | Phosphodiester cyclique du dihydroxy-5,6 indole | >260° |
| 28 | Thiocarbonyldioxy-5,6 indole | >260° |

TABLEAU I suite

| No. | Désignation du composé | Pt de fusion et d'ébullition (E) °C |
|---|---|---|
| 29 | Méthoxy-5 triméthylsilyloxy-6 indole | 90—1° |
| 30 | Di(triméthylsilyloxy)-5,6 méthyl-2 indole | 99° |
| 31 | Carbonyldioxy-5,6 méthyl-2 indole | 143° |
| 32 | Hydroxy-(5 ou 6)myristoyloxy-(6 ou 5)indole | 125—126° |
| 33 | Dimyristoyloxy-5,6 indole | 92° |
| 34 | Hydroxy-(5 ou 6)oléoyloxy-(6 ou 5)indole | 94—100° |
| 35 | Dioléoyloxy-5,6 indole | 38—40° |
| 36 | Di(triméthylsilyloxy)-5,6 carbéthoxy-2 indole | 143° |
| 37 | Di(triméthylsilyloxy)-5,6 triméthylsilyloxy carbonyl-2 indole | 173° |
| 38 | Di(triméthylsilyloxy)-5,6 méthyl-3 indole | 84° |
| 39 | Hexadécoxy-6 méthoxy-5 indole | 68,5° |
| 40 | Hexadécoxy-6 hydroxy-5 indole | 80° |
| 41 | Hexadécoxy-5 hydroxy-6 indole | 79° |
| 42 | Dipivaloyoxy-5,6 indole | 139° |
| 43 | Hydroxy-(5 ou 6)pivaloyloxy-(6 ou 5)indole | 130° |
| 44 | Dihexanoyloxy-5,6 indole | 60°—63° |
| 45 | Hexanoyloxy-(5 ou 6)hydroxy-(6 ou 5)indole | 88° |
| 46 | Dibutanoyloxy-5,6 indole | 74° |
| 47 | Butanoyloxy-(5 ou 6)hydroxy-(6 ou 5)indole | 121° |

* à 1,06 × $10^2$ Pa

Les composés destinés à être utilisés selon l'invention plus particulièrement préférés répondent à la formule I dans laquelle $R_1$ représente hydrogène, $R_2$ et $R_3$ identiques ou différents, représentent un atome d'hydrogène ou un groupement alkyle inférieur, l'un au moins des substituants $R_4$ ou $R_5$ représente un groupement alkyle en $C_1$—$C_{20}$ linéaire ou ramifié ou un groupement acyle en $C_2$—$C_{20}$ linéaire ou ramifié, un groupement alcénoyle en $C_3$—$C_{20}$, linéaire ou ramifié, l'autre représentant hydrogène ou encore $R_4$ et $R_5$ représentant simultanément $SiR_9R_{10}R_{11}$ où $R_9$, $R_{10}$, $R_{11}$ ont les significations indiquées ci-dessus.

La composition utilisée conformément à l'invention pour la coloration de la peau contiennent dans un milieu cosmétiquement acceptable et approprié pour une application topique, les composés de formule (I) définis ci-dessus.

Les composés de formule (I) sont présents de préférence dans des compositions conformes à l'invention dans les proportions comprises entre 0,01 et 15% en poids par rapport au poids total de la composition.

Le milieu cosmétique approprié pour une application sur la peau est soit aqueux, soit anhydre. Il peut contenir un ou plusieurs solvants acceptables sur le plan cosmétique. Parmi ces solvants, on peut citer à titre d'exemples les alcanols inférieurs en $C_1$—$c_4$ tels que l'éthanol, l'isopropanol, ou le t-butanol, l'éthylèneglycol, le propylèneglycol, le dipropylèneglycol et le ter-propylèneglycol, les éthers monométhylique, monoéthylique et monobutylique de l'éthylène glycol et l'acétate du monoéthyléther de l'éthylèneglycol.

Les compositions conformes à l'invention peuvent se présenter sous forme de lotions, d'huiles, de gels, d'émulsions, de crèmes, de laits, de sticks ou de baumes.

Les lotions peuvent être des lotions hydroalcooliques ou des lotions oléoalcooliques à base d'alcools inférieurs tels que l'éthanol ou d'un glycol tel que le propylèneglycol et d'esters d'acides gras tels que le myristate d'isopropyle.

Le milieu cosmétique approprié est de préférence sous forme anhydre. On appelle milieu anhydre au sens de l'invention, un milieu contenant moins de 1% d'eau.

Le milieu cosmétique conforme à l'invention peut également contenir ou être constitué exclusivement de corps gras qui peuvent être choisis parmi les huiles minérales telles que l'huile de vaseline; les huiles animales telles que les huiles de baleine, de phoque, de menhaden, de foie de flétan, de morue, de thon, de tortue, de suif, de pied de boeuf, de pied de cheval, de pied de mouton, de vison, de loutre, de marmotte, etc.; les huiles végétales telles que les huiles d'amande, d'arachide, de colza, de germes de blé, d'olive, de germes de maïs, de jojoba, de sésame, de tournesol, d'olive de palme, de noix ou des huiles similaires.

Parmi les corps gras, on peut également citer la vaseline, la paraffine, la lanoline hydrogénée, la lanoline acétylée, les huiles de silicone. Les cires qui peuvent être utilisées peuvent être entre autres, la cire de sipol, la cire de lanoline, la cire d'abeille, la cire de candellila, le cire microcristalline, la cire de Carbauba, la cire de spermaceti, le beurre de cacao, le beurre de karité, les cires de silicone, les huiles hydrogénées concrètes à 25°C, les sucroglycérides, les oléates, les myristates, les linoléates, les stéarates de calcium, de magnésium, de zirconium ou d'aluminium. Des alcools gras pouvant être utilisés dans de telles compositions et sont, entre autres, les alcools laurique, cétylique, myristique, stéarique, palmitique et oléique, éventuellement polyoxyéthylénés ou polyglycérolés. A titre d'alcools gras polyoxyéthylénés, on peut citer les alcools laurique, cétylique, stéarylique et oléique comportant de 2 à 20 moles d'oxyde d'éthylène.

Les compositions selon l'invention peuvent bien entendu contenir des adjuvants cosmétiques habituellement utilisés dans ce type de compositions tels que des épaississants, des adoucissants, des humectants, de surgraissants, des séquestrants, des émollients, des mouillants, des agents tensio-actifs, des polymères, des conservateurs, des antimousses, des parfums, des émulsionnants, des bactéricides ou tout autre ingrédient habituellement utilisé en cosmétique.

Les formes de réalisation particulièrement préférées sont constituées par les huiles anhydres et les sticks.

Les compositions conformes à l'invention peuvent être utilisées pour la protection ou l'entretien du bronzage et contenir en plus du composé de formule (I) des filtres solaires spécifiques du rayonnement UV-B et/ou du rayonnement UVA et compatibles avec les composés destinés à être utilisés conformément à l'invention. On peut donc obtenir une composition filtrant l'ensemble des rayonnements UV-B et UV-A ou seulement l'une des fractions de ces rayonnements tout en procurant une coloration de la peau analogue à la pigmentation résultant du bronzage naturel.

L'invention a également pour objet un procéde de coloration de la peau analogue à la pigmentation résultant d'un bronzage naturel et mettant en oeuvre un composé répondant à la formule I.

Les composés destinés à être utilisés conformément à l'invention sont connus en eux-mêmes pour certains et nouveaux pour d'autres. Les composés nouveaux répondent à la formule:

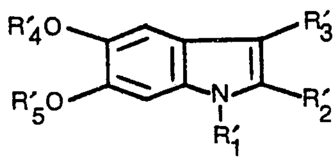

(II)

dans laquelle $R'_1$ représente un atome d'hydrogène ou un groupement alkyle inférieur; $R'_2$ et $R'_3$ identiques ou différents représentent un atome d'hydrogène, un groupement alkyle inférieur, un groupement carboxyle, un groupement alcoxycarbonyle inférieur ou un groupement $-COOSi(CH_3)_3$; $R'_4$ et $R'_5$, identiques ou différents, peuvent représenter un groupement alkyle, linéaire ou ramifié en $C_9-C_{20}$, un groupement acyle, linéaire ou ramifié, en $C_{10}-C_{20}$, un groupement alcénoyle, linéaire ou ramifié, en $C_3-C_{20}$, un groupement $-P(O)(OR_6)_2$, l'autre groupement $R'_4$ ou $R'_5$ pouvant être un atome d'hydrogène, un groupement alkyle en $C_1-C_8$, un groupement formyle, un groupement acyle en $C_2-C_9$ ou un groupement aralkyle;

$R'_4$ ou $R'_5$ pouvant désigner un groupement $-Si(CH_3)_3$ lorsque $R'_1$ est différent de méthyle; ou bien $R'_4$ et $R'_5$ avec les atomes d'oxygène auxquels ils sont rattachés, forment un cycle contenant éventuellement un groupement carbonyle quand l'un des substituants $R'_1$, $R'_2$ ou $R'_3$ est différent de l'hydrogène, un groupement thiocarbonyle, un groupement $>P(O)OR_6$ ou $>CR_7R_8$, $R_6$ désignant un atome d'hydrogène, un radical alkyle inférieur et $R_7$ représentant un atome d'hydrogène, un groupement alkyle inférieur et $R_8$ représentant un groupement alcoxy inférieur ou un groupement mono ou dialkylamino et les sels correspondant avec des métaux alcalins, alcalino-terreux ou d'amines.

Les produits destinés à être utilisés conformément à l'invention peuvent être préparés suivant des procédés connus en eux-mêmes. Les dérivés du dihydroxy-5,6 indole substitués ou non en 2 et/ou 3

peuvent être synthétisés à partir de composés déjà substitués en 5 et en 6, la dernière étape de formation étant une cyclisation réductrice d'un dérivé β-2-dinitrostyrène:

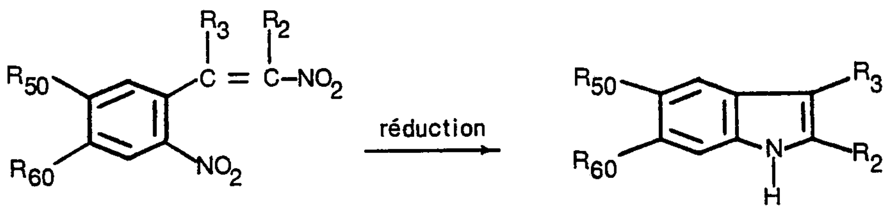


ou bien les dérivés du dihydroxy-5,6 indole substitués en 1,2 et/ou 3 peuvent être synthétisés à partir des dihydroxy-5,6 indole substitués en 1,2 et/ou 3 par des méthodes dans lesquelles on évite la présence de bases libres dans le mélange réactionnel dû à l'instabilité en milieu basique des dihydroxy-5,6 indole substitués en 1,2 et/ou 3.

Par ce fait, on peut travailler, soit par transfert de phase dans le cas des éthérifications (tel que pour les composés 39, 40 et 41), soit par des méthodes de transestérification dans le cas des esters des dihydroxy-5,6 indole substitués en 1,2 ou/et 3 (tel que pour les composés 27, 28, 31—35). Dans ces derniers cas, les dérivés monoacylés et diacylés sont séparés par colonne chromatographique.

Les exemples suivants sont destinés à illustrer les composés et compositions destinés à être utilisés selon l'invention.

Le tableau II regroupe les spectres d'absorption de certains composés destinés à être utilisés selon l'invention, les numéros faisant référence à ceux figurant au tableau I.

TABLEAU II

Spectres d'absorption UV

| No. | Solvant | λmax (ε max) |
|---|---|---|
| 3 | Ethanol | 295 nm (7180)<br>272 nm (5120) |
| 4 | Propylèneglycol | 300 nm (5360)<br>275 nm (3780)<br>230 nm (4590) |
| 5 | Propylèneglycol | 300 nm (4020)<br>282 nm (ep.)<br>275 nm (4750)<br>230 nm (6800) |
| 6 | Ethanol | 283 nm (6500) |
| 8 | Ethanol | 296 nm (6500)<br>274 nm (4920) |
| 19 | Propylèneglycol | 296 nm (5120)<br>288 nm (4800)<br>228 nm (7320) |
| 21 | Ethanol | 302 nm (3960)<br>274 nm (2950) |
| 23 | Ethanol | 301 nm (6010)<br>274 nm (3990) |
| 25 | Ethanol | 300 nm (3800)<br>276 nm (ep. 2950) |
| 26 | Ethanol | 305 nm (6480)<br>274 nm (3750) |

TABLEAU II suite

Spectres d'absorption UV

| No. | Solvant | λmax (ε max) |
|---|---|---|
| 28 | Ethanol | 312 nm (24100)<br>263 nm (8750) |
| 29 | Ethanol | 298 nm (6100)<br>274 nm (4400) |
| 30 | Ethanol | 300 nm (8300)<br>275 nm (5520) |
| 31 | Ethanol | 301 nm (8170)<br>278 nm (ep. 5370)<br>219 nm (20900) |
| 32 | Ethanol | 296 nm (6500)<br>273 nm (4860) |
| 33 | Ethanol | 284 nm (6870)<br>220 nm (29500) |
| 34 | Ethanol | 296 nm (6730)<br>273 nm (5050) |
| 35 | Ethanol | 284 nm (7000) |
| 36 | Ethanol | 317 nm (22000) |
| 39 | Ethanol | 297 nm (5800)<br>273 nm (3900) |
| 40 | Ethanol | 300 nm (5990)<br>273 nm (4000) |
| 41 | Ethanol | 300 nm (6500)<br>273 nm (4170) |
| 42 | Ethanol | 284 nm (6900) |
| 43 | Ethanol | 295 nm (6340)<br>273 nm (4700) |
| 44 | Ethanol | 284 nm (7100) |
| 45 | Ethanol | 295 nm (6400)<br>273 nm (4910) |
| 46 | Ethanol | 284 nm (6600) |
| 47 | Ethanol | 295 nm (6400)<br>273 nm (4880) |

## EXEMPLES DE PREPARATION

### Exemple I

Préparation du composé no. 17 du tableau I (Formyloxy-5 hydroxy-6 indole et formyloxy-6 hydroxy-5 indole)

A 1,8 g (0,0122 mole) de dihydroxy-5,6 indole en solution dans 20 ml d'éther sec, on ajoute goutte à

goutte à −5°C, 2,39 g (0,0257 mole) d'anhydride formyl acétique. En 6 heures, on remonte progressivement la température à 20°C. On laisse une nuit sous agitation. On recueille un précipité blanc que l'on filtre et recristallise dans un mélange toluène-acétone (3/2). On obtient le composé *17*: 218 mg (rendement: 10%) sous forme d'une poudre blanche. Le composé *17* est un mélange 60/40 des deux formylés comme indiqué par le spectre RMN du proton. MS (70 eV) pour $C_9H_7NO_3$: 177 ($M^+$, 58%) 149 (100), 120 (17), 103 (45), et 65 (21).

Exemple II

Préparation du compose no. 18 du tableau I (Acétoxy-6 formyloxy-5 indole et acétoxy-5 formyloxy-6 indole)

Le dérivé précédent *17* (150 mg, $8,5 \times 10^{-4}$ mole) est agité 4 heures avec 2,25 ml d'anhydride acétique et 0,12 ml de pyridine. Après évaporation des solvants, reprise dans le dichlorométhane et lavages successifs avec des solutions aqueuses de HCl 0,IN, $NaHCO_3$ 2% et eau, séchage, on obtient le dérive *18*: 150 mg, (rendement: 80%) qui, d'après le spectre RMN, est un mélange 60/40 des deux isomères.

Exemple III

Préparation du composé no. 19 du tableau I (Formyloxy-6 méthoxy-5 indole)

On porte au reflux pendant 16 heures sous azote une solution de 12 g (0,0735 mole) d'hydroxy-6 méthoxy-5 indole et 12,95 g (0,147 mole) d'anhydride formyl acétique dans 100 ml de toluène. Après refroidissement, on ajoute 30 g de Silice 60 au milieu réactionnel, on filtre et on concentre de moitié le filtrat. Le précipité blanc obtenu est filtré puis recristallisé à nouveau dans 30 ml de toluène. Après séchage sous vide, on obtient 4,2 g du dérivé *19* (poudre blanche, rendement 30%).

Analyse: $C_{10}H_9NO_3$

Calculé: C 62,82; H 4,74; N 7,33

Trouvé: C 62,84; H 4,75; N 7,29.

Exemple IV

Préparation du compose no. 25 du tableau I [di(triméthyl silyloxy)]-5,6 indole

A 0,81 g (0,004 mole) de N,O-bis (triméthylsilyl) acétamide, on ajoute en agitant à température ambiante 0,3 g (0,002 mole) de dihydroxy-5,6 indole. Quand la dissolution est totale, on ajoute 2 ml de dichlorométhane et passe cette solution sur une colonne de silice 60 en éluant du dichlorométhane. La première fraction obtenue est concentrée au rotavapeur puis séchée sous vide. On obtient ainsi 0,42 g (rendement: 71%) de cristaux blancs du dérive *25.*

Analyse: $C_{10}H_9NO_3Si_2$

Calculé: C 57,29; H 7,90; N 4,77

Trouvé: C 57,39; H 7,96; N 4,72.

Exemple V

Préparation du composé no. 26 du tableau I [(Ethoxy-1 éthyl)-1,1 dioxy]-5,6 indole

Dans un ballon équipé d'une rampe de distillation, on chauffe 14,6 ml (0,08 mole) de triéthylorthoacétate et 3 g (0,02 mole) de dihydroxy-5,6 indole sur un bain d'huille porté à 120°C de telle sorte que l'éthanol formé distille en continu (temps de réaction: 4 heures). On distille l'excès de triéthylorthoacétate et isole la fraction de point d'ébullition 160°C à $1,06 \times 10^2$ Pa. L'huile incolore obtenue cristallise pour donner le composé *26* (2,71 g, rendement: 62%).

Analyse: $C_{12}H_{13}NO_3$

Calculé: C 65,74; H 5,98; N 6,39

Trouvé: C 65,34; H 6,01; N 6,29.

Exemple VI

Préparation du composé no. 24 du tableau I

Hydroxy-5(triméthylsilyloxy)-6 indole et hydroxy-6(triméthyl silyloxy)-5 indole

On agite 2 heures et demie une solution de 200 ml de tetrahydrofuranne (THF) sec contenant 3 g (0,02 mole) de dihydroxy-5,6 indole et 8,2 g (0,04 mole) de bis(triméthylsilyl)urée. Après addition de 100 ml de toluène, on lave à l'eau la phase organique et après séchage sur sulfate de sodium on la concentre sous vide. Les 5 g de résidu sont passés rapidement sur colonne de silice (éluant: $CH_2Cl_2$). On récupère à côté du dérivé disilylé *25*, 0,5 g du dérivé *24* (rendement: 11%), un mélange 30/70 des deux monosilylés comme indiqué par le spectre RMN du proton.

Analyse: $C_{11}H_{15}NO_2Si$

Calculé: C 59,69; H 6,83; N 6,33

Trouvé: C 59,27; H 6,86; N 6,33.

Exemple VII

Préparation du composé no. 27 du tableau I (Phosphodiester cyclique du dihydroxy-5,6 indole)

A une solution de 4,14 g (0,06 mole) de triazole-1,2,4 et 1,84 ml (0,02 mole) d'oxychlorure de phosphore dans 150 ml de dioxane sec, on ajoute, à température ambiante, sous azote et en absence d'humidité, 6,06 g (0,06 mole) de triéthylamine en 15 minutes. On laisse agiter 40 minutes à 20°C. On filtre le

chlorhydrate de triéthylamine formé en évitant les contacts du filtrat avec l'air. La solution de phosphoryl tris(triazole-1,2,4) obtenue est ajoutée en 2 heures à 20°C sous azote à une solution de 2,68 g (0,018 mole) de dihydroxy-5,6 indole. On agite ensuite 3 heures et demie, on laisse reposer une nuit et on filtre le précipité obtenu (2,9 g sec). Ce précipité est agité 1 heure à température ambiante dans 100 ml d'au, filtré à nouveau et séché. On récupère le dérivé *27* (1 g, rendement: 26%).

Le spectre RMN est conforme à la structure attendue.

Exemple VIII

Préparation du composé no. 28 du tableau I (Thiocarbonyldioxy-5,6 indole)

A une solution de 1,49 g (0,01 mole) de dihydroxy-5,6 indole dans 100 ml d'éther isopropylique et 50 ml de toluène, on ajoute, goutte à goutte à 60°C et sous azote, une solution de 2,82 g (0,0158 mole) de thiocarbonyldiimidazole dans 400 ml de toluène. On laisse agiter 2 heures à 60°C. Le mélange réactionnel est concentré sous vide. Au résidu obtenu, on ajoute 200 ml d'eau. Le précipité jaune clair est filtré, lavé abondamment à l'eau. On le redissout dans 50 ml d'acétone et le reprécipite par 300 ml d'eau. Après séchage sous vide de 13,3 Pa, on obtient 1,2 g (rendement: 63%) du dérive *28* (poudre légèrement jaune).

Analyse: $C_9H_5NO_2S$

Calculé: C 56,56; H 2,64; N 7,33; S 16,77

Trouvé: C 56,57; H 2,58; N 7,19; S 16,64.

Exemple IX

Préparation du composé no. 20 du tableau I (Benzyloxy-6 butoxy-5 indole)

On chauffe 2 heures au reflux sous agitation un mélange de butoxy-5 hydroxy-4 nitro-2 benzaldéhyde (31 g, 0,13 mole), de chlorure de benzyle (20,2 g, 0,16 mole) et de carbonate de potassium (22,11 g, 0,16 mole) dans 80 ml de diméthylformamide. On verse le mélange réactionnel dans 200 ml d'eau glacée et filtre le précipité. On obtient après recristallisation dans un mélange hexane-toluène le benzyloxy-4 butoxy-5 nitro-2 benzaldéhyde (30,2 g, rendement 71%, poudre jaune); fusion = 94°C.

A un mélange du dérivé précédent (26,3 g, 0,08 mole) dans 90 ml d'acide acétique glacial et d'acétate d'ammonium sec (8,85 g 0,115 mole) on ajoute du nitrométhane (10,1 ml, 0,185 mole). Après 5 heures de reflux, on verse le mélange réactionnel dans 200 ml d'eau glacée. On filtre le précipité brun et on le recristallise dans l'éthanol. On obtient le benzyloxy-4 butoxy-5 dinitrio-2, β-styrène (18,4 g rendement 62%, poudre jaune); fusion = 152°C.

Une solution de 160 ml d'éthanol absolu et 80 ml d'acide acétique est portée à 60°C. A cette solution, on ajoute du fer activé (48 g); on porte le mélange à 80—85°C sous bonne agitation et on ajoute le dérivé précédemment obtenu (8,9 g, 0,024 mole) en 15 minutes. Après 30 minutes d'agitation à 85°C, on filtre les boues ferriques, on les rince avec 300 ml d'acide acétique puis 300 ml d'éthanol. On dilue le filtrat avec de la glace. Le précipité formé est filtré, lavé à l'eau et séché. Après passage sur une colonne de Silice 60 (éluant: $CH_2Cl_2$) on récupère le dérivé *20* (5 g, rendement 70%).

Analyse: $C_{19}H_{21}NO_2$

Calculé: C 77,26; H 7,16; N 4,74

Trouvé: C 77,36; H 7,17; N 4,72.

Exemple X

Préparation du compose no. 21 du tableau I (Butoxy-5 hydroxy-6 indole)

Le dérivé *20* précédent (4 g, 0,0135 mole) a été hydrogéné sous 50 atmosphères d'hydrogène dans une bombe avec 40 ml d'éthanol et 0,6 g de palladium à 10% sur charbon pendant 3 heures. Après filtration, évaporation du solvant, le résidu a été recristallisé dans un mélange benzène-hexane pour donner le dérivé *21* (2,5 g, rendement 90%).

Analyse: $C_{12}H_{15}NO_2$

Calculé: C 70,22; H 7,37; N 6,82

Trouvé: C 70,31; H 7,28; N 6,77.

Exemple XI

Préparation du composé no. 22 du tableau I (Benzyloxy-5 butoxy-6 indole)

A une solution de 28 ml d'éthanol absolu et 14 ml d'acide acétique, on ajoute à 60°C 8,6 g de fer activé. On maintient 15 minutes à 80° et ajoute le benzyloxy-5 butoxy-4 dinitro-2, β-styrène (1,6 g, 0,0043 mole). On laisse à 80° pendant 1 heure et filtre les boues ferriques que l'on rince avec 40 ml d'éthanol et 40 ml d'acide acétique. On dilue le filtrat avec 100 ml d'eau glacée. Après extraction de la solution avec du chlorure de méthylène, séchage sur sulfate de sodium de la phase organique, celle-ci est concentrée et chromatographiée sur silice 60 (éluant toluène/$CH_2Cl_2$ 50:50). On obtient 0,5 g (rendement 40%) du dérivé *22*.

Analyse: $C_{19}H_{21}NO_2$

Calculé: C 77,26; H 7,16; N 4,74

Trouvé: C 77,06; H 7,14; N 4,82.

Exemple XII

Préparation du composé no. 23 du tableau I (Butoxy-6 hydroxy-5 indole)

Le dérivé *22* précédent (0,5 g, 0,0017 mole) a été hydrogéné sous 50 atmosphères d'hydrogène dans une bombe avec 5 ml d'éthanol absolu et 70 mg de palladium sur charbon à 10% pendant 2 heures. Après filtration et évaporation du solvant, le résidu a été purifié par chromatographie sur silice 60 (éluant: $CH_2Cl_2$) pour donner le dérivé *23* (0,19 g, poudre beige, rendement 55%).

Analyse: $C_{12}H_{12}NO_2$

Calculé: C 70,22; H 7,37; N 6,82

Trouvé: C 70,11; H 7,37; N 6,75.

Exemple XIII

Préparation du composé no. 29 du tableau I (Méthoxy-5 triméthylsilyloxy-6 indole)

On mélange à température ordinaire le dérivé *4* (2,04 g, 0,0125 mole) et le N,O-bis(triméthylsilyl)acétamide (5,08 g, 0,025 mole) jusqu'à complète solubilisation. Après chromatographie sur colonne de silice 60 (éluant: $CH_2Cl_2$) on obtient le dérivé *29* (2,54 g, rendement 86%).

Analyse: $C_{12}H_{17}NO_2Si$

Calculé: C 61,24; H 7,28; N 5,95

Trouvé: C 61,30; H 7,32; N 6,01.

Exemple XIV

Préparation du composé no. 30 du tableau I (Di(triméthylsilyloxy)-5,6 méthyl-2 indole)

Le dérivé *14* (90 mg, $5,5 \times 10^{-4}$ mole) et le N,O-bis(triméthylsilyl)acétamide (220 mg, $1,1 \times 10^{-3}$ mole) sont agités à température ordinaire jusqu'à solubilisation complète. Le produit obtenu est purifié sur colonne de silice 60 (éluant: toluène/$CH_2Cl_2$ 50:50). On obtient le dérivé *30* (0,14 g, rendement 83%).

Analyse: $C_{15}H_{25}NO_2Si_2$

Calculé: C 58,58; H 8,19; N 4,55

Trouvé: C 58,54; H 8,16; N 4,60.

Exemple XV

Préparation du composé no. 31 du tableau I (Carbonyldioxy-5,6 méthyl-2 indole)

Au dérivé *14* (0,51 g, 0,0031 mole) dissous dans 50 ml d'éther isopropylique, on ajoute au reflux le carbonyldiimidazole (1,67 g, 0,0103 mole) en solution dans 300 ml de toluène. Après deux heures au reflux, on ajoute 200 ml d'eau et sépare la phase toluénique qui est ensuite séchée sur sulfate de sodium. Le solvant est évaporé et le produit recristallisé dans un mélange 50:50 d'eau et d'éthanol. On obtient le dérivé *31* (0,45 g, rendement 76%).

Analyse: $C_{10}H_7NO_3$

Calculé: C 63,49; H 3,73; N 7,40

Trouvé: C 63,44; H 3,75; N 7,06.

Exemples XVI et XVII

Préparation des composés no. 32 et 33 du tableau I (Hydroxy-(5 ou 6)myristoyloxy-(6 ou 5)indole et dimyristoyloxy-5,6 indole)

On porte au reflux pendant 4 heures une solution de dihydroxy-5,6 indole (2,98 g, 0,02 mole) dans 300 ml de THF et du N-myristoylimidazole (5,57 g, 0,02 mole). Après concentration sous vide, le résidu est chromatographié sur colonne de silice 60 (éluant: $CH_2Cl_2$) pour donner le composé *33* (2,6 g, rendement 23%).

Analyse: $C_{36}H_{59}NO_4$

Calculé: C 75,88; H 10,44; N 2,46

Trouvé: C 75,60; H 10,40; N 2,71.

et le composé *32* (2,7 g, rendement 38%), lequel est un mélange 70/30 des deux monoesters comme indiqué par le spectre RMN du proton.

Analyse: $C_{22}H_{33}NO_3$

Calculé: C 73,50; H 9,25; N 3,90

Trouvé: C 73,59; H 9,25; N 3,87.

Exemples XVIII et XIX

Préparation des composés no. 34 et 35 du tableau I (Hydroxy-(5 ou 6) oléoyloxy-(6 ou 5)indole et dioléoyloxy-5,6 indole

Dans une solution de dihydroxy-5,6 indole (2,98 g, 0,02 mole) dans 50 ml de tétrahydrofuranne, on ajoute goutte à goutte, à 20°C et sous azote, du N-oléylimidazole en solution dans 100 ml de tétrahydrofuranne. On porte au reflux pendant 4 heures. Le solvant est éliminé sous vide et le résidu est

chromatographié sur silice 60 (éluant: toluène/$CH_2Cl_2$ 50:50) pour donner le composé *35* (1,9 g, rendement 14%).

Analyse: $C_{44}H_{71}NO_4$
Calculé: C 77,94; H 10,55; N 2,06
Trouvé: C 77,84; H 10,34; N 2,11.

et le composé *34* (éluant $CH_2Cl_2$) (5 g, rendement 60%), lequel est un mélange 70/30 des deux monoesters comme indiqué par le spectre RMN du proton.

Analyse: $C_{26}H_{39}NO_3$
Calculé: C 75,50; H 9,50; N, 3,39
Trouvé: C 75,62; H 9,54; N 3,41.

## Exemple XX

Préparation du composé no. 36 du tableau I (Di(triméthylsilyloxy)-5,6 carbéthoxy-2 indole)

Le dérivé *11* (442 mg, 0,002 mole) et le N,O-bis(triméthylsilyl)acétamide (814 mg, 0,004 mole) sont agités à 40°C pendant 30 minutes. La solution obtenue est chromatographiée sur silice 60 (éluant: toluène/$CH_2Cl_2$ 50:50). Après concentration sous vide, on obtient le dérivé *36* (0,59 g, rendement 73%).

Analyse: $C_{17}H_{27}NO_4Si_2$
Calculé: C 55,85; H 7,44; N 3,83
Trouvé: C 55,90; H 7,55; N 3,77.

## Exemple XXI

Préparation du composé no. 37 du tableau I (Di(triméthylsilyloxy)-5,6 triméthylsilyloxycarbonyl-2 indole)

Le dérivé *12* (0,2 g, 0,00103 mole) et le N,O-bis(triméthylsilyl)acétamide (2,5 g, 0,0124 mole) sont portés à 60°C pendant 1 heure sous agitation. La solution est versée dans 50 g de glace et le précipité est filtré et lavé à l'eau. Il est repris dans le dichlorométhane et séché sur sulfate de sodium. Après évaporation sous vide, on obtient le dérivé *37* (0,35 g, rendement 83%).

MS (70 eV) pour $C_{18}H_{31}NO_4Si_3$: 409 ($M^+$, 12,5) 337 (41), 319 (14), 232 (30), 75 (75) et 73 (100).

## Exemple XXII

Préparation du composé no. 38 du tableau I (Di(triméthylsilyloxy)-5,6 méthyl-3 indole)

Le dérivé *16* (0,3 g, 0,0018 mole) et le N,O-bis(triméthylsilyl)acétamide (0,61 g, 0,0036 mole) sont agités pendant 30 minutes à température ordinaire. La solution obtenue est chromatographiée sur silice 60 (éluant: $CH_2Cl_2$) pour donner le dérivé *38* (0,43 g, rendement 78%).

Analyse: $C_{15}H_{25}NO_2Si_2$
Calculé: C 58,57; H 8,19; N 4,55
Trouvé: C 58,55; H 8,18; N 4,53.

## Exemple XXIII

Préparation du composé no. 39 du tableau I (Hexadécoxy-6 méthoxy-5 indole)

On introduit, goutte à goutte, en 20 minutes, le dérivé *4* (2 g, 0,0123 mole) dissous dans 10 ml de diméthylformamide (DMF) dans un mélange de bromo-1 hexadécane (4,5 g, 0,0148 mole) et de carbonate de potassium (1,87 g, 0,0135 mole) dans 45 ml de DMF à 70°C et sous azote. Le mélange réactionnel est agité sous azote à 80°C pendant 3 heures et demie. Le mélange noirâtre est versé dans de l'eau glacée sous agitation et le solide brunâtre formé est immédiatement filtré et lavé à l'eau. Il est repris dans du dichlorométhane et séche sur sulfate de sodium. Après chromatographie sur silice 60 (éluant: toluène/$CH_2Cl_2$ 50:50), et recristallisation dans l'hexane, on obtient le dérivé *39* (1,66 g, rendement 35%).

Analyse: $C_{25}H_{41}NO_2$
Calculé: C 77,47; H 10,66; N 3,61
Trouvé: C 77,35; H 10,62; N 3,71.

## Exemples XXIV et XXV

Préparation des composés no. 40 et 41 du tableau I (Hexadécoxy-6 hydroxy-5 indole et hexadécoxy-5 hydroxy-6 indole)

Dans 30 ml de DMF sec, on introduit successivement à 50°C sous azote et sous agitation, du carbonate de potassium (2,76 g, 0,02 mole), du dihydroxy-5,6 indole (3 g, 0,02 mole) et du bromo-1 hexadécane (6,14 g, 0,02 mole). On chauffe à 60—70°C pendant 2 heures et demie. Le mélange noirâtre est coulé dans de l'eau glacée sous vive agitation et le précipité marron foncé est immédiatement filtré, lavé à l'eau, repris au dichlorométhane et séché 5 minutes sur sulfate de sodium. La solution est concentrée et chromatographiée sur silice 60 (éluant: toluène/$CH_2Cl_2$ 50:50) pour donner dans les fractions 14 à 18 le composé *40* (1,8 g, rendement 24%).

Analyse: $C_{24}H_{39}NO_2$
Calculé: C 77,16; H 10,52; N 3,75
Trouvé: C 77,44; H 10,54; N 3,85.

et dans les fractions 23 à 33, le composé *41* (0,8 g, rendement 11%).

Analyse: $C_{24}H_{39}NO_2$

| | | | |
|---|---|---|---|
| Calculé: | C 77,16; | H 10,52; | N 3,75 |
| Trouvé: | C 77,07; | H 10,59; | N 3,91. |

Exemples XXVI et XXVII

Préparation des composés no. 42 et 43 du tableau I [Dipivaloyloxy-5,6 indole et hydroxy-(5 ou 6)pivaloyloxy-(6 ou 5) indole]

A une solution d'acide pivalique (1,122 g, 0,011 mole) dans 25 ml de chlorure de méthylène, on ajoute du N,N'-carbonyl diimidazole (1,78 g, 0,011 mole). On laisse sous agitation à température ordinaire jusqu'à ce que le dégagement gazeux de $CO_2$ cesse (1 heure). Sous azote et à température ordinaire, on ajoute le dihydroxy-5,6 indole (1,64 g, 0,011 mole) et on laisse sous agitation pendant 4 heures. La phase organique est lavée à l'eau et séchée sur sulfate de sodium. Après séparation sur colonne chromatographique de silice 60, on obtient le dérivé *42* (éluant: toluène/$CH_2Cl_2$ 50:50) (0,59 g, rendement 17%).

Analyse: $C_{18}H_{23}NO_4$

| | | | |
|---|---|---|---|
| Calculé: | C 68,12; | H 7,30; | N 4,41 |
| Trouvé: | C 68,02; | H 7,27; | N 4,49 |

et le dérive *43* (éluant: $CH_2Cl_2$) (1,74 g, rendement 68%).

Analyse: $C_{13}H_{15}NO_3$

| | | | |
|---|---|---|---|
| Calculé: | C 66,94; | H 6,48; | N 6,00 |
| Trouvé: | C 66,77; | H 6,49; | N 5,90. |

Exemples XXVIII et XXIX

Préparation des composés no. 44 et 45 du tableau I [Dihexanoyloxy-5,6 indole et hexanoyloxy-(5 ou 6)hydroxy-(6 ou 5)indole]

De manière identique aux exemples précédents, l'acide hexanoïque (2,65 g, 0,022 mole) a été traité dans le chlorure de méthylène (50 ml) avec le N,N'-carbonyl diimidazole (3,75 g, 0,022 mole) et le dihydroxy-5,6 indole (3,28 g, 0,022 mole). On obtient le dérivé *44* (1,90 g, rendement 25%).

Analyse: $C_{20}H_{27}NO_4$

| | | | |
|---|---|---|---|
| Calculé: | C 69,54; | H 7,89; | N 4,05 |
| Trouvé: | C 69,26; | H 7,93; | N 3,96 |

et le dérivé *45* (3,21 g, rendement 59%)

Analyse: $C_{14}H_{17}NO_3$

| | | | |
|---|---|---|---|
| Calculé: | C 68,00; | H 6,93; | N 5,66 |
| Trouvé: | C 67,65; | H 6,98; | N 5,64. |

Exemples XXX et XXXI

Préparation des composés no. 46 et 47 du tableau I

Dibutanoyloxy-5,6 indole et butanoyoxy-(5 ou 6)hydroxy-(6 ou 5) indole

De manière identique aux exemples précédents, l'acide butanoïque (2,9 g, 0,033 mole) a été traité dans le chlorure de méthylène (75 ml) avec le N,N'-carbonyl diimidazole (5,35 g, 0,033 mole) et le dihydroxy-5,6 indole (4,92 g, 0,033 mole). On obtient le dérivé *46* (2,77 g, rendement 29%).

Analyse: $C_{16}H_{19}NO_4$

| | | | |
|---|---|---|---|
| Calculé: | C 66,42; | H 6,62; | N 4,84 |
| Trouvé: | C 66,65; | H 6,64; | N 4,76 |

et le dérivé *47* (2,64 g, rendement 36,5%).

Analyse: $C_{12}H_{13}NO_3$

| | | | |
|---|---|---|---|
| Calculé: | C 65,74; | H 5,98; | N 6,39 |
| Trouvé: | C 65,46; | H 5,94; | N 6,14. |

Exemple de Composition 1

On prépare la composition suivante:

| | | |
|---|---|---|
| Composé *4* | | 0,75 g |
| Alcool oléocétylique à 30 moles d'oxyde d'éthylène | | 7,0 g |
| Alcool stéarylique | | 4,0 g |
| Myristate d'isopropyle | | 4,0 g |
| Huile de vaseline | | 11,0 g |
| Huile de silicone | | 1,0 g |
| Propylèneglycol | | 5,0 g |
| Sorbitol en solution aqueuse à 70% | | 10,0 g |
| Conservateur, parfums | qs | |
| Eau déminéralisée | qsp | 1000 g |

Cette composition se présente sous la forme d'un lait. Appliquée sur la peau, elle permet après exposition à la lumière solaire, d'obtenir une coloration gris-brun proche de la pigmentation naturelle.

Exemple de Composition 2

On prépare la composition huileuse suivante:

| | | |
|---|---|---|
| Composé *25* | | 0,15 g |
| Monobutyléther de l'éthylèneglycol | | 10,0 g |
| Myristate d'isopropyle | | 20,0 g |
| Huile de tournesol | | 11,0 g |
| Huile de silicone | qsp | 100 g |

Après application sur la peau de cette composition huileuse, suivie d'une exposition au soleil, on obtient une coloration brunâtre.

Exemple de Composition 3

On prépare la composition suivante:

| | | |
|---|---|---|
| Composé *25* | | 0,1 g |
| Cire minérale d'hydrocarbures vendue sous la dénomination de OZECIRE 310 S par le Societété CIRES ET DERIVES | | 15,0 g |
| Cire d'abeilles | | 6,0 g |
| Triglycérides d'acide ricinoléique | | 15,0 g |
| Alcool oléique | | 12,0 g |
| Lanoline hydrogénée vendue sous la dénomination de HYDROLAN H par la Société ONYX | | 8,0 g |
| Lanoline liquide vendue sous la dénomination de ARGONOL 60 par la Société WESTBROOK | | 7,0 g |
| Cire de Carnauba | | 1,5 g |
| Triglycérides hydrogénés et interestérifiés (origine palme et coprah) vendus sous la dénomination de LIPOCIRE A par la Société GATTEFOSSE | | 5,0 g |
| Ditertiobutyl paracrésol | | 0,1 g |
| Huile de vaseline | qsp | 100 g |

Cette composition permet la préparation de sticks. L'application sur la peau, suivie d'une exposition au soleil, conduit à une coloration gris-brun.

Exemple de Composition 4

On prépare la composition suivante:

| | | |
|---|---|---|
| Composé *4* | | 0,5 g |
| Polymère carboxyvinylique vendu sous la dénomination de CARBOPOL 940 par la Société GOODRICH | | 0,4 g |
| Prolylèneglycol | | 5,0 g |
| Myristate d'isopropyle | | 3,0 g |
| Mélange de mono et distéarate de glycérol vendu sous la dénomination de GELEOL par la Société GATTEFOSSE | | 2,0 g |
| Alcool éthylique | | 20,0 g |
| Triéthanolamine | | 0,6 g |
| Conservateur | qs | |
| Eau déminéralisée | qsp | 100 g |

Cette composition se présente sous forme d'un gel opaque. L'application sur la peau et l'exposition au soleil conduit à une coloration gris-brun.

Exemple de Composition 5

On prépare la composition suivante:

| | | |
|---|---|---|
| Composé *4* | | 0,9 g |
| Sorbitol en solution aqueuse à 70% | | 5,0 g |
| Mélange de mono et distéarate de glycérol vendu sous la dénomination de GELEOL par la Société GATTEFOSSE | | 2,0 g |
| Alcool myristique | | 6,0 g |
| Alcool oléique à 20 moles d'oxyde d'éthylène | | 10,0 g |
| Myristate d'isopropyle | | 4,0 g |
| Huile de silicone | | 1,0 g |
| Huile de tournesol | | 12,0 g |
| Conservateur, parfums | qs | |
| Eau déminéralisée | qsp | 100 g |

Cette composition est une crème. L'application sur la peau, suivie d'une exposition au soleil, permet l'obtention d'une coloration gris-brun similaire à la pigmentation résultant d'un bronzage naturel.

Exemple de Composition 6

On prépare la composition suivante:

| | | |
|---|---|---|
| Composé *8* | | 0,1 g |
| Propylèneglycol | | 5,0 g |
| Alcool éthylique | | 5,0 g |
| Hydroxyéthylcellulose vendu sous la dénomination de CELLOSIZE PCG10 par la Société UNION CARBIDE | | 1,5 g |
| Eau déminéralisée | qsp | 100 g |

Cette composition se présente sous forme de gel. L'application sur la peau, suivie d'une exposition au soleil, permet l'obtention d'une coloration gris-brun.

**Revendications**

1. Utilisation pour la coloration de la peau d'un composé répondant à la formule:

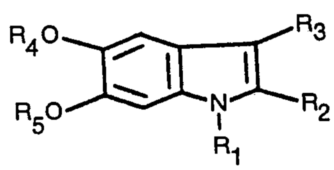

(I)

dans laquelle $R_1$ représente un atome d'hydrogène, un groupement alkyle inférieur ayant 1 à 6 atomes de carbone, ou un reste $—SiR_9R_{10}R_{11}$, $R_2$ et $R_3$ identiques ou différents, représentent un atome d'hydrogène, un groupement alkyle inférieur ayant 1 à 6 atomes de carbone, un groupement carboxyle, un groupement

alcoxy ($C_1$—$C_6$) carbonyle inférieur ou un groupement —$COOSiR_9R_{10}R_{11}$; $R_4$ et $R_5$, identiques ou différents, représentent un atome d'hydrogène, un groupement alkyle linéaire ou ramifié en $C_1$—$C_{20}$, un groupement formyle, un groupement acyle linéaire ou ramifiée en $C_2$—$C_{20}$, un groupement alcénoyle linéaire ou ramifié en $C_3$—$C_{20}$, un groupement $R_6OSO_2$—, un groupement —$SiR_9R_{10}R_{11}$, un groupement —$P(O)(OR_6)_2$, un groupement aralkyle, ou bien $R_4$ et $R_5$, avec les atomes d'oxygène auxquels ils sont liés, forment un cycle contenant éventuellement un groupement carbonyle quand l'un au moins des groupements $R_1$, $R_2$ ou $R_3$ est différent d'hydrogène ou bien un groupement thiocarbonyle ou un reste >$P(O)OR_6$, un groupement >$CR_7R_8$ ou un groupement méthylène, $R_6$ représentant un atome d'hydrogène ou un groupement alkyle inférieur ayant 1 à 6 atomes de carbone, $R_7$ représentant un atome d'hydrogène ou un groupement alkyle inférieur ayant 1 à 6 atomes de carbone, $R_8$ représentant un groupement alcoxy inférieur ayant 1 à 6 atomes de carbone ou un groupement mono ou dialkylamino et $R_9$, $R_{10}$ ou $R_{11}$, identiques ou différents, représentant des groupements alkyle inférieurs ayant 1 à 6 atomes de carbone, linéaires ou ramifiés, l'un au moins des substituants $R_1$, $R_2$, $R_3$, $R_4$, $R_5$ étant différent de l'hydrogène; et les sels correspondants avec les métaux alcalins, alcalino-terreux ou d'amines.

2. Utilisation pour conférer à la peau une coloration sensiblement analogue à la pigmentation résultant du bronzage naturel, d'une composition cosmétique, caractérisée par le fait qu'elle contient, dans un milieu cosmétiquement acceptable et approprié pour une application topique, au moins un composé de formule:

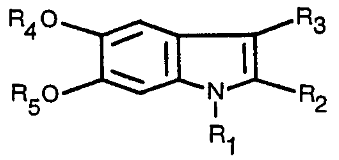

(I)

dans laquelle $R_1$ représente un atome d'hydrogène, un groupement alkyle inférieur ayant 1 à 6 atomes de carbone, ou un reste —$SiR_9R_{10}R_{11}$, $R_2$ et $R_3$ identiques ou différents représentent un atome d'hydrogène, un groupement alkyle inférieur ayant 1 à 6 atomes de carbone, un groupement carboxyle, un groupement alcoxy ($C_1$—$C_6$) carbonyle ou un groupement —$COOSiR_9R_{10}R_{11}$; $R_4$ et $R_5$, identiques ou différents, représentent un atome d'hydrogène, un groupement alkyle linéaire ou ramifié en $C_1$—$C_{20}$, un groupement formyle, un groupement acyle linéaire ou ramifiée en $C_2$—$C_{20}$, un groupement alcénoyle linéaire ou ramifié en $C_3$—$C_{20}$, un groupement $R_6OSO_2$—, un groupement —$SiR_9R_{10}R_{11}$, un groupement —$P(O)(OR_6)_2$, un groupement aralkyle, ou bien $R_4$ et $R_5$, avec les atomes d'oxygène auxquels ils sont liés, forment un cycle contenant éventuellement un groupement carbonyle quand l'un au moins des groupements $R_1$, $R_2$ ou $R_3$ est différent d'hydrogène ou bien un groupement thiocarbonyle, un reste >$P(O)OR_6$, un groupement >$CR_7R_8$ ou un groupement méthylène, $R_6$ représentant un atome d'hydrogène ou un groupement alkyle inférieur ayant 1 à 6 atomes de carbone, $R_7$ représentant un atome d'hydrogène ou un groupement alkyle inférieur ayant 1 à 6 atomes de carbone, $R_8$ représentant un groupement alcoxy inférieur ayant 1 à 6 atomes de carbone ou un groupement mono ou dialkylamino et $R_9$, $R_{10}$ ou $R_{11}$, identiques ou différents, représentant des groupements alkyle inférieurs ayant 1 à 6 atomes de carbone, linéaires ou ramifiés, l'un au moins des substituants $R_1$, $R_2$, $R_3$, $R_4$, $R_5$ étant différent de l'hydrogène; et les sels correspondants avec les métaux alcalins, alcalino-terreux ou d'amines.

3. Utilisation selon la revendication 1 ou 2, caractérisée par le fait qu'elle met en oeuvre un composé répondant à la formule (I) dans laquelle $R_1$ représente hydrogène, $R_2$ et $R_3$, identiques ou différents, représentent un atome d'hydrogène ou un groupement alkyle inférieur ayant 1 à 6 atomes de carbone, l'un au moins des groupements $R_4$ ou $R_5$ représente un groupement alkyle en $C_1$—$C_{20}$ linéaire ou ramifiée, un groupement acyle en $C_2$—$C_{20}$, linéaire ou ramifié, ou un groupement alcénoyle en $C_3$—$C_{20}$, linéaire ou ramifié, l'autre substituant étant hydrogène ou encore $R_4$ et $R_5$ représentent simultanément $SiR_9R_{10}R_{11}$, dans laquelle $R_9$, $R_{10}$, $R_{11}$ ont les significations indiquées dans la revendication 1.

4. Utilisation selon la revendication 1 ou 2, caractérisée par le fait que les composés sont choisis parmi les dibenzyloxy-5,6 indole, benzyloxy-5 méthoxy-6 indole, benzyloxy-6 méthoxy-5 indole, hydroxy-6 méthoxy-5 indole, hydroxy-5 méthoxy-6 indole, diacétoxy-5,6 indole, acétoxy-6 méthoxy-5 indole, acétoxy-(5 ou 6)hydroxy-(6 ou 5)indole, dibenzyloxy-5,6 carbéthoxy-2 indole, dibenzyloxy-5,6 carboxy-2 indole, dihydroxy-5,6 carbéthoxy-2 indole, dihydroxy-5,6 carboxy-2 indole, dibenzyloxy-5,6 méthyl-2 indole, dihydroxy-5,6 méthyl-2 indole, dibenzyloxy-5,6 méthyl-3 indole, dihydroxy-5,6 méthyl-3 indole, formyloxy-(5 ou 6)hydroxy-(6 ou 5) indole, acétoxy-(5 ou 6) formyloxy-(6 ou 5)indole, formyloxy-6 méthoxy-5 indole, benzyloxy-6 butoxy-5 indole, butoxy-5 hydroxy-6 indole, benzyloxy-5 butoxy-6 indole, butoxy-6 hydroxy-5 indole, hydroxy-(5 ou 6)triméthylsilyloxy-(6 ou 5)indole, di(triméthylsilyloxy)-5,6 indole, [(éthoxy-1 éthyl)-1,1 dioxy]-5,6 indole, phosphodiester cyclique du dihydroxy-5,6 indole, thiocarbonyldioxy-5,6 indole, méthoxy-5 triméthylsilyloxy-6 indole, di(triméthylsilyloxy)-5,6 méthyl-2 indole, carbonyldioxy-5,6 méthyl-2 indole, hydroxy-(5 ou 6)myristoyloxy-(6 ou 5)indole, dimyristoyloxy-5,6 indole, hydroxy-(5 ou 6)oléyloxy-(6 ou 5)indole, dioléyloxy-5,6 indole, di(triméthylsilyloxy)-5,6 carbéthoxy-2 indole, di(triméthylsilyloxy)-5,6 triméthylsilyloxycarbonyl-2 indole, di(triméthylsilyloxy)-5,6 méthyl-3 indole, hexadécoxy-6 méthoxy-5 indole, hexadécoxy-6 hydroxy-5 indole, hexadecoxy-5 hydroxy-6 indole, dipivaloyloxy-5,6 indole, hydroxy-(5 ou 6)pivaloyloxy-(6 ou 5) indole, dihexanoyloxy-5,6 indole, hexanoyloxy-(5 ou 6)hydroxy-(6 ou 5)indole, dibutanoyloxy-5,6 indole, butanoyloxy-(5 ou 6)hydroxy-(6 ou 5)indole.

5. Utilisation selon l'une quelconque des revendications 2 à 4, caractérisée par le fait que le milieu cosmétique de la composition est constitué par un milieu anhydre.

6. Utilisation selon l'une quelconque des revendications 2 à 5, caractérisée par le fait que le milieu cosmétique de la composition contient au moins un solvant choisi parmi les alcanols inférieurs, l'éthylèneglycol, le propylèneglycol, le dipropylèneglycol, le terpropylèneglycol, les éthers monométhylique, monoéthylique et monobutylique de l'éthylène glycol et l'acétate du monoéthyléther de l'éthylèneglycol.

7. Utilisation selon l'une quelconque des revendications 2 à 6, caractérisée par le fait que le milieu cosmétique de la composition contient ou est constitué exclusivement de corps gras.

8. Utilisation selon l'une quelconque des revendications 2 à 7, caractérisée par le fait que la composition se présente sous la forme d'une lotion, d'un gel, d'une émulsion, d'un baume, d'un stick, d'une huile, d'un lait ou d'une crème.

9. Utilisation selon l'une quelconque des revendications 1 à 8, caractérisée par le fait que la composition contient en plus, des adjuvants cosmétiques habituellement utilisés dand les compositions topiques choisis parmi les épaississants, les adoucissants, les humectants, les surgraissants, les séquestrants, les émollients, les mouillants, les agents tensio-actifs, les polymères, les conservateurs, les agents antimousse, les parfums, les émulsionnants, les bactéricides et tout autre ingrédient habituellement utilisé en cosmétique.

10. Utilisation selon l'une quelconque des revendications 8 à 9, en vue d'assurer de façon complémentaire pour la protection ou l'entretien du bronzage, caractérisée par le fait que la composition contient en plus, des filtres solaires spécifiques du rayonnement UV-B et/ou du rayonnement UV-A et compatibles avec les composés de formule (I).

11. Utilisation selon l'une quelconque des revendications 2 à 10, caractérisée par le fait que les composés de formule (I) sont présents dans la composition dans les proportions comprises entre 0,01 et 15% en poids par rapport au poids total de la composition.

12. Procédé de coloration de la peau afin de lui conférer une coloration sensiblement analogue à la pigmentation résultant du bronzage naturel caractérisé par le fait que l'on applique sur la peau au moins un composé de formule (I) selon la revendication 1, dans un support cosmétique approprié.

13. Composition cosmétique destinée à conférer à la peau une coloration sensiblement analogue à la pigmentation résultant du bronzage naturel, caractérisée par le fait qu'elle contient, dans un milieu cosmétiquement acceptable et approprié pour une application topique, au moins un composé de formule:

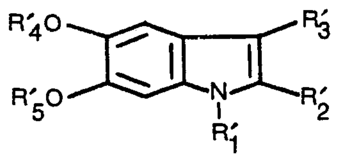

(II)

dans laquelle $R'_1$ représente un atome d'hydrogène ou un groupement alkyle inférieur; $R'_2$ et $R'_3$, identiques ou différents représentent un atome d'hydrogène, un groupement alkyle inférieur, un groupement carboxyle, un groupement alcoxycarbonyle inférieur ou un groupement $-COOSi(CH_3)_3$; $R'_4$ et $R'_5$, identiques ou différents, peuvent représenter un groupement alkyle, linéaire ou ramifié en $C_9-C_{20}$, un groupement acyle, linéaire ou ramifié, en $C_{10}-C_{20}$, un groupement alcénoyle, linéaire ou ramifié, en $C_3-C_{20}$, un groupement $-P(O)(OR_6)_2$, l'autre groupement $R'_4$ ou $R'_5$ pouvant être un atome d'hydrogéne, un groupement alkyle en $C_1-C_8$, un groupement formyle, un groupement acyle en $C_2-C_9$ ou un groupement aralkyle;

$R'_4$ ou $R'_5$ pouvant désigner un groupement $-Si(CH_3)_3$ lorsque $R'_1$ est différent de méthyle; ou bien $R'_4$ et $R'_5$ avec les atomes d'oxygène auxquels ils sont rattachés, forment un cycle contenant éventuellement un groupement carbonyle quand l'un des substituants $R'_1$, $R'_2$ ou $R'_3$ est différent de l'hydrogène, un groupement thiocarbonyle, un groupement $>P(O)OR_6$ ou $>CR_7R_8$, $R_6$ désignant un atome d'hydrogène, un radical alkyle inférieur et $R_7$ représentant un atome d'hydrogène, un groupement alkyle inférieur et $R_8$ représentant un groupement alcoxy inférieur ou un groupement mono ou dialkylamino et les sels correspondant avec des métaux alcalins, alcalino-terreux ou d'amines.

14. Composition cosmétique destinée à conférer à la peau une coloration similaire au bronzage naturel, caractérisée par le fait qu'elle contient au moins un composé choisi parmi les dibenzyloxy-5,6 indole, benzyloxy-5 méthoxy-6 indole, benzyloxy-6 méthoxy-5 indole, hydroxy-6 méthoxy-5 indole, hydroxy-5 méthoxy-6 indole, acétoxy-6 methoxy-5 indole, acétoxy-(5 ou 6) hydroxy-(6 ou 5)indole, dibenzyloxy-5,6 carbéthoxy-2 indole, dibenzyloxy-5,6 carboxy-2 indole, dihydroxy-5,6 carbéthoxy-2 indole, dibenzyloxy-5,6 méthyl-2 indole, dibenzyloxy-5,6 méthyl-3 indole, formyloxy-(5 ou 6)hydroxy-(6 ou 5) indole, acétoxy-(5 ou 6)formyloxy-(6 ou 5)indole, formyloxy-6 méthoxy-5 indole, benzyloxy-6 butoxy-5 indole, butoxy-5 hydroxy-6 indole, benzyloxy-5 butoxy-6 indole, butoxy-6 hydroxy-5 indole, hydroxy-(5 ou 6) triméthylsilyloxy-(6 ou 5)indole, di(triméthylsilyloxy)-5,6 indole, [(éthoxy-1 éthyl)-1,1 dioxy]-5,6 indole, phosphodiester cyclique du dihydroxy-5,6 indole, thiocarbonyldioxy-5,6 indole, méthoxy-5 triméthylsilyloxy-6 indole, di(triméthylsilyloxy)-5,6 méthyl-2 indole, carbonyldioxy-5,6 méthyl-2 indole, hydroxy-(5

ou 6)myristoyloxy-(6 ou 5)indole, dimyristoyloxy-5,6 indole, hydroxy-(5 ou 6)oléyloxy-(6 ou 5)indole, dioléyloxy-5,6 indole, di(triméthylsilyloxy)-5,6 carbéthoxy-2 indole, di(triméthylsilyloxy)-5,6 triméthylsilyloxycarbonyl-2 indole, di(triméthylsilyloxy)-5,6 méthyl-3 indole, hexadécyloxy-6 méthoxy-5 indole, hexadécyloxy-6 hydroxy-5 indole, hexadecyloxy-5 hydroxy-6 indole, dipivaloyloxy-5,6 indole, hydroxy-(5 ou 6)pivaloyloxy-(6 ou 5) indole, dihexanoyloxy-5,6 indole, hexanoyloxy-(5 ou 6)hydroxy-(6 ou 5)indole, dibutanoyloxy-5,6 indole, butanoyloxy-(5 ou 6)hydroxy-(6 ou 5)indole.

**Patentansprüche**

1. Verwendung einer Verbindung der Formel sowie deren Alkali-, Erdalkalimetallsalze oder Amine

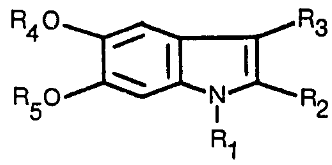

(I)

worin $R_1$ ein Wasserstoffatom, eine Niedrigalkylgruppe mit 1 bis 6 Kohlenstoffatomen oder einen Rest $—SiR_9R_{10}R_{11}$, $R_2$ und $R_3$, gleich oder verschieden, Wasserstoff, eine Niedrigalkylgruppe mit 1 bis 6 Kohlenstoffatomen, eine Carboxylgruppe, eine $(C_1—C_6)$-Alkoxycarbonylgruppe oder eine Gruppe $—COOSiR_9R_{10}R_{11}$, $R_4$ und $R_5$, gleich oder verschieden, ein Wasserstoffatom, eine lineare oder verzweigte $C_1—C_{20}$-Alkylgruppe, Formylgruppe, lineare oder verzweigte $C_2—C_{20}$-Acylgruppe, lineare oder verzweigte $C_3—C_{20}$-Alkenoylgruppe, eine Gruppe $R_6OSO_2—$, eine Gruppe $—SiR_9R_{10}R_{11}$, eine Gruppe $—P(O)(OR_6)_2$, eine Aralkylgruppe bedeuten oder $R_4$ und $R_5$ mit den Sauerstoffatomen, an die sie gebunden sind, einen Ring bilden, der gegebenenfalls eine Carbonylgruppe, wenn mindestens einer der Gruppen $R_1$, $R_2$ oder $R_3$ von Wasserstoff verschieden ist, oder einen Thiocarbonylgruppe, einen Rest $=P(O)OR_6$, eine Gruppe $=CR_7R_8$ oder eine Methylengruppe enthält, wobei $R_6$ ein Wasserstoffatom oder eine Niedrigalkylgruppe mit 1 bis 6 Kohlenstoffatomen, $R_7$ eine Wasserstoffatom oder eine Niedrigalkylgruppe mit 1 bis 6 Kohlenstoffatomen, $R_8$ eine Niedrigalkoxygruppe mit 1 bis 6 Kohlenstoffatomen oder eine Mono- oder Dialkylaminogruppe und $R_9$, $R_{10}$ oder $R_{11}$, gleich oder verschieden, lineare oder verzweigte Niedrigalkylgruppen mit 1 bis 6 Kohlenstoffatomen darstellen, und worin mindestens einer der Substituenten $R_1$, $R_2$, $R_3$, $R_4$, $R_5$ von Wasserstoff verschieden ist, zur Hautfärbung.

2. Verwendung einer kosmetischen Zusammensetzung, um der Haut eine Färbung zu verleihen, die der aus der natürlichen Bräunung entstehenden Pigmentierung in etwa analog ist, dadurch gekennzeichnet, daß die Zusammensetzung in einem Medium, das kosmetisch verträglich und zur örtlichen Aufbringung geeignet ist, mindestens eine Verbindung der Formel sowie deren Alkali-, Erdalkalimetallsalze oder Amine enthält:

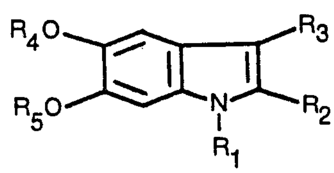

(I)

worin $R_1$ ein Wasserstoffatom, eine Niedrigalkylgruppe mit 1 bis 6 Kohlenstoffatomen oder einen Rest $—SiR_9R_{10}R_{11}$, $R_2$ und $R_3$, gleich oder verschieden, Wasserstoff, eine Niedrigalkylgruppe mit 1 bis 6 Kohlenstoffatomen, eine Carboxylgruppe, eine $(C_1—C_6)$-Alkoxycarbonylgruppe oder eine Gruppe $—COOSiR_9R_{10}R_{11}$, $R_4$ und $R_5$, gleich oder verschieden, ein Wasserstoffatom, eine lineare oder verzweigte $C_1—C_{20}$-Alkylgruppe, Formylgruppe, lineare oder verzweigte $C_2—C_{20}$-Acylgruppe, lineare oder verzweigte $C_3—C_{20}$-Alkenoylgruppe, eine Gruppe $R_6OSO_2—$, eine Gruppe $—SiR_9R_{10}R_{11}$, eine Gruppe $—P(O)(OR_6)_2$, eine Aralkylgruppe bedeuten oder $R_4$ und $R_5$ mit den Sauerstoffatomen, an die sie gebunden sind, einen Ring bilden, der gegebenenfalls eine Carbonylgruppe, wenn mindestens einer der Gruppen $R_1$, $R_2$ oder $R_3$ von Wasserstoff verschieden ist, oder einen Thiocarbonylgruppe, einen Rest $=P(O)OR_6$, eine Gruppe $=CR_7R_8$ oder eine Methylengruppe enthält, wobei $R_6$ ein Wasserstoffatom oder eine Niedrigalkylgruppe mit 1 bis 6 Kohlenstoffatomen, $R_7$ eine Wasserstoffatom oder eine Niedrigalkylgruppe mit 1 bis 6 Kohlenstoffatomen, $R_8$ eine Niedrigalkoxygruppe mit 1 bis 6 Kohlenstoffatomen oder eine Mono- oder Dialkylaminogruppe und $R_9$, $R_{10}$ oder $R_{11}$, gleich oder verschieden, lineare oder verzweigte Niedrigalkylgruppen mit 1 bis 6 Kohlenstoffatomen darstellen, und worin mindestens einer der Substituenten $R_1$, $R_2$, $R_3$, $R_4$, $R_5$ von Wasserstoff verschieden ist, zur Hautfärbung.

3. Verwendung gemäß Anspruch 1 oder 2, dadurch gekennzeichnet, daß man eine Verbindung der Formel (I) einsetzt, worin $R_1$ Wasserstoff, $R_2$ und $R_3$, gleich oder verschieden, ein Wasserstoffatom oder eine Niedrigalkylgruppe mit 1 bis 6 Kohlenstoffatomen oder eine Niedrigalkylgruppe mit 1 bis 6 Kohlenstoffatomen, mindestens eine der Gruppen $R_4$ oder $R_5$ eine lineare oder verzweigte $C_1$—$C_{20}$-Alkylgruppe, eine lineare oder verzweigte $C_2$—$C_{20}$-Acylgruppe oder eine lineare oder verzweigte $C_3$—$C_{20}$-Alkenoylgruppe darstellen, wobei der andere Substituent Wasserstoff ist, oder $R_4$ und $R_5$ gleichzeitig $SiR_9R_{10}R_{11}$ bedeuten, worin $R_9$, $R_{10}$, $R_{11}$ die in Anspruch 1 angegebenen Bedeutungen haben.

4. Verwendung gemäß Anspruch 1 oder 2, dadurch gekennzeichnet, daß die Verbindungen ausgewählt sind unter Dibenzyloxy-5,6-indol, Benzyloxy-5methoxy-6indol, Benzyloxy-6methoxy-5indol, Hydroxy-6methoxy-5indol, Hydroxy-5methoxy-6indol, Diacetoxy-5,6indol, Acetoxy-6methoxy-5indol, Acetoxy-(5 oder 6)hydroxy-(6 oder 5)indol, Dibenzyloxy-5,6carbethoxy-2indol, Dibenzyloxy-5,6carboxy-2indol, Dihydroxy-5,6carbethoxy-2indol, Dihydroxy-5,6carboxy-2indol, Dibenzyloxy-5,6methyl-2indol, Dihydroxy-5,6methyl-2indol, Dibenzyloxy-5,6methyl-3indol, Dihydroxy-5,6methyl-3indol, Formyloxy-(5 oder 6)hydroxy-(6 oder 5)indol, Acetoxy-(5 oder 6)formyloxy-(6 oder 5)indol, Formyloxy-6methoxy-5indol, Benzyloxy-6butoxy-5indol, Butoxy-5hydroxy-6indol, Benzyloxy-5butoxy-6indol, Butoxy-6hydroxy-5indol, Hydroxy-(5 oder 6)trimethylsilyloxy-(6 oder 5)indol, Di(trimethylsylyloxy)-5,6indol, ((Ethoxy-1-ethyl)-1,1dioxy)-5,6indol, zyklischer Phosphodiester von Dihydroxy-5,6indol, Thiocarbonyldioxy-5,6indol, Methoxy-5trimethylsilyloxy-6indol, Di(trimethylsilyloxy)-5,6methyl-2indol, Carbonyldioxy-5,6methyl-2indol, Hydroxy-(5 oder 6)myristoyloxy-(6 oder 5)indol, Dimyristoyloxy-5,6indol, Hydroxy-(5 oder 6) oleyloxy-(6 oder 5)indol, Dioleyloxy-5,6indol, Di(trimethylsilyloxy)-5,6carbethoxy-2indol, Di(trimethylsilyloxy)-5,6trimethylsilyloxycarbonyl-2indol, Di(trimethylsilyloxy)-5,6methyl-3indol, Hexadecoxy-6methoxy-5indol Hexadecoxy-6hydroxy-5indol, Hexadecoxy-5hydroxy-6indol, Dipivaloyoxy-5,6indol, Hydroxy-(5 oder 6)pivaloyloxy-(6 oder 5)indol, Dihexanoyloxy-5,6indol, Hexanoyloxy-(5 oder 6) hydroxy-(6 oder 5)indol, Dibutanoyloxy-5,6indol, Butanoyloxy-(5 oder 6)hydroxy-(6 oder 5)indol.

5. Verwendung gemäß jedem der Ansprüche 2 bis 4, dadurch gekennzeichnet, daß das kosmetische Medium der Zusammensetzung aus einem wasserfreien Medium zusammengesetzt ist.

6. Verwendung gemäß jedem der Ansprüche 2 bis 5, dadurch gekennzeichnet, daß das kosmetische Medium der Zusammensetzung mindestens ein Lösungsmittel enthält, ausgewählt unter Niedrigalkanol, Ethylenglycol, Propylenglycol, Dipropylenglycol, Terpropylenglycol, Monomethyl-, Monoethyl- und Monobutylethylenglycol und dem Acetat des Monoethylethers von Ethylenglycol.

7. Verwendung gemäß jedem der Ansprüche 2 bis 6, dadurch gekennzeichnet, daß das kosmetische Medium der Zusammensetzung Fettkörper enthält oder ausschließlich daraus zusammengesetzt ist.

8. Verwendung gemäß jedem der Ansprüche 2 bis 7, dadurch gekennzeichnet, daß die Zusammensetzung sich unter der Form einer Lotion, eines Gels, einer Emulsion, einer Salbe, eines Stifts, eines Öls, einer Milch oder einer Creme darstellt.

9. Verwendung gemäß jedem der Ansprüche 2 bis 8, dadurch gekennzeichnet, daß die Zusammensetzung zusätzlich kosmetische Hilfsstoffe enthält, die in örtlich wirkenden Zusammensetzungen üblicherweise eingesetzt werden, ausgewählt unter Verdickungsmitteln, Linderungsmitteln, Benetzungsmitteln, Schmiermitteln, Beaufschlagungsmitteln, Weichmachern, Befeuchtungsmitteln, oberflächenaktiven Mitteln, Polymeren, Konservierungsmitteln, Antischaummitteln, Parfüm, Emulgatoren, Bakteriziden sowie einem jeden anderen Bestandteil, der üblicherweise in der Kosmetik verwendet wird.

10. Verwendung gemäß jedem der Ansprüche 8 und 9, dadurch gekennzeichnet, daß zur ergänzenden Sicherung von Schutz und Pflege der Bräunung die Zusammensetzung zusätzlich Sonnenfilter enthält, die für UV-B- und/oder UV-A-Bestrahlung spezifisch und mit den Verbindungen der Formel (I) verträglich sind.

11. Verwendung gemäß jedem der Ansprüche 2 bis 10, dadurch gekennzeichnet, daß die Verbindungen der Formel (I) in der Zusammensetzung in Anteilen zwischen 0,01 und 15 Gewichtsprozent, bezogen auf das Gesamtgewicht der Zusammensetzung, vorhanden sind.

12. Verfahren zur Färbung der Haut, um ihr eine Färbung zu verleihen, die der aus der natürlichen Bräunung entstehenden Pigmentierung in etwa analog ist, dadurch gekennzeichnet, daß man auf die Haut mindestens eine Verbindung der Formel (I) gemäß Anspruch 1 in einem kosmetisch geeigneten Träger aufbringt.

13. Kosmetische Zusammensetzung, die dazu bestimmt ist, der Haut eine Färbung zu verleihen, die der aus der natürlichen Bräunung entstehenden Pigmentierung in etwa analog ist, dadurch gekennzeichnet, daß sie in einem Medium, das kosmetisch verträglich und zur örtlichen Aufbringung geeignet ist, mindestens eine Verbindung der Formel sowie deren Alkali-, Erdalkalimetallsalze oder Amine enthält:

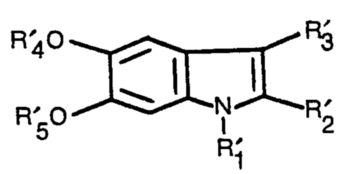

(II)

worin $R'_1$ ein Wasserstoffatom, eine Niedrigalkylgruppe, $R'_2$ und $R'_3$, gleich oder verschieden, Wasserstoff, Niedrigalkyl, Carboxyl, Niedrigalkoxycarbonyl oder eine Gruppe $—COOSi(CH_3)_3$ darstellen; $R'_4$ und $R'_5$, gleich oder verschieden, lineares oder verzweigtes $C_9$—$C_{20}$-Alkyl, lineares oder verzweigtes $C_{10}$—$C_{20}$-Acyl, lineares oder verzweigtes $C_3$—$C_{20}$-Alkenoyl, eine Gruppe $—P(O)(OR_6)_2$, bedeuten können, wobei die andere Gruppe $R'_4$ oder $R'_5$ Wasserstoff, $C_1$—$C_8$-Alkyl, Formyl, $C_2$—$C_9$-Acyl oder eine Aralkylgruppe sein können,

$R'_4$ oder $R'_5$ eine Grüppe —$Si(CH_3)_3$ darstellen kann, wenn $R'_1$ verschieden von Methyl ist, oder $R'_4$ und $R'_5$ mit den Sauerstoffatomen, an die sie gebunden sind, einen Ring bilden, der gegebenenfalls eine Carbonylgruppe, wenn einer der Substituenten $R'_1$, $R'_2$ oder $R'_3$ verschieden von Wasserstoff ist, eine Thiocarbonylgruppe, eine Gruppe $=P(O)OR_6$ oder $=CR_7R_8$ enthält, wobei $R_6$ Wasserstoff oder ein Niedrigalkyl, $R_7$ Wasserstoff oder ein Niedrigalkyl und $R_8$ eine Niedrigalkoxygruppe oder eine Mono- oder Dialkylaminogruppe darstellen.

14. Kosmetische Zusammensetzung, die dazu bestimmt ist, der Haut eine der natürlichen Bräunung ähnliche Färbung zu verleihen, dadurch gekennzeichnet, daß sie mindestens eine Verbindung enthält, ausgewählt unter Dibenzyloxy-5,6-indol, Benzyloxy-5methoxy-6indol, Benzyloxy-6methoxy-5indol, Hydroxy-6methoxy-5indol, Hydroxy-5methoxy-6indol, Diacetoxy-5,6indol, Acetoxy-6methoxy-5indol, Acetoxy-(5 oder 6)hydroxy-(6 oder 5)indol, Dibenzyloxy-5,6carbethoxy-2indol, Dibenzyloxy-5,6carboxy-2indol, Dihydroxy-5,6carbethoxy-2indol, Dihydroxy-5,6carboxy-2indol, Dibenzyloxy-5,6methyl-2indol, Dihydroxy-5,6methyl-2indol, Dibenzyloxy-5,6methyl-3indol, Dihydroxy-5,6methyl-3indol, Formyloxy-(5 oder 6)hydroxy-(6 oder 5)indol, Acetoxy-(5 oder 6)formyloxy-(6 oder 5)indol, Formyloxy-6methoxy-5indol, Benzyloxy-6butoxy-5indol, Butoxy-5hydroxy-6indol, Benzyloxy-5butoxy-6indol, Butoxy-6hydroxy-5indol, Hydroxy-(5 oder 6)trimethylsilyloxy-(6 oder 5)indol, Di(trimethylsilyloxy)-5,6indol, ((Ethoxy-1-ethyl)-1,1dioxy)-5,6indol, zyklischer Phosphodiester von Dihydroxy-5,6indol, Thiocarbonyldioxy-5,6indol, Methoxy-5trimethylsilyloxy-6indol, Di(trimethylsilyloxy)-5,6methyl-2indol, Carbonyldioxy-5,6methyl-2indol, Hydroxy-(5 oder 6)myristoyloxy-(6 oder 5)indol, Dimyristoyloxy-5,6indol, Hydroxy-(5 oder 6) oleyloxy-(6 oder 5)indol, Dioleyloxy-5,6indol, Di(trimethylsilyloxy)-5,6carbethoxy-2indol, Di(trimethylsilyloxy)-5,6trimethylsilyloxycarbonyl-2indol, Di(trimethylsilyloxy)-5,6methyl-3indol, Hexadecoxy-6methoxy-5indol Hexadecoxy-6hydroxy-5indol, Hexadecoxy-5hydroxy-6indol, Dipivaloyoxy-5,6indol, Hydroxy-(5 oder 6)pivaloyloxy-(6 oder 5)indol, Dihexanoyloxy-5,6indol, Hexanoyloxy-(5 oder 6)hydroxy-(6 oder 5)indol, Dibutanoyloxy-5,6indol, Butanoyloxy-(5 oder 6)hydroxy-(6 oder 5)indol.

**Claims**

1. Use for colouring skin of a compound corresponding to the formula:

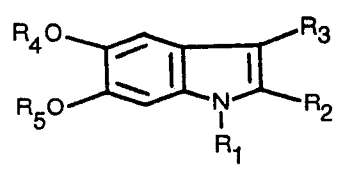

(I)

in which $R_1$ represents a hydrogen atom, a lower alkyl group having 1 to 6 carbon atoms, or a residue $—SiR_9R_{10}R_{11}$, $R_2$ and $R_3$, which may be identical or different, represent a hydrogen atom, a lower alkyl group having 1 to 6 carbon atoms, a carboxyl group, a lower ($C_1$—$C_6$) alkoxycarbonyl group or a group $—COOSiR_9R_{10}R_{11}$; $R_4$ and $R_5$, which may be identical or different, represent a hydrogen atom, a linear or branched $C_1$—$C_{20}$ alkyl group, a formyl group, a linear or branched $C_2$—$C_{20}$ acyl group, a linear or branched $C_3$—$C_{20}$ alkenoyl group, a group $R_6OSO_2$—, a group $—SiR_9R_{10}R_{11}$, a group $—P(O)(OR_6)_2$, an aralkyl group, or alternatively $R_4$ and $R_5$, with the oxygen atoms to which they are attached, form a ring optionally containing a carbonyl group when at least one of the groups $R_1$, $R_2$ or $R_3$ is other than hydrogen, or alternatively a thiocarbonyl group, a residue $>P(O)OR_6$, a group $>CR_7R_8$ or a methylene group, $R_6$ representing a hydrogen atom or a lower alkyl group having 1 to 6 carbon atoms, $R_7$ representing a hydrogen atom or a lower alkyl group having 1 to 6 carbon atoms, $R_8$ representing a lower alkoxy group having 1 to 6 carbon atoms or a mono- or dialkylamino group and $R_9$, $R_{10}$ or $R_{11}$, which may be identical or different, representing linear or branched lower alkyl groups having 1 to 6 carbon atoms, at least one of the substituents $R_1$, $R_2$, $R_3$, $R_4$ and $R_5$ being other than hydrogen; and the corresponding salts with alkali metals, alkaline earth metals or amines.

2. Use, for imparting to the skin a coloration substantially similar to the pigmentation resulting from natural tanning, of a cosmetic composition, characterized in that it contains, in a cosmetically acceptable medium suitable for a topical application, at least one compound of formula (I)

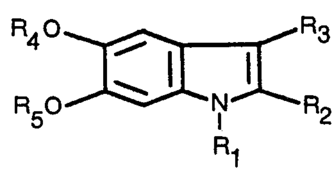

(I)

in which $R_1$ represents a hydrogen atom, a lower alkyl group having 1 to 6 carbon atoms, or a residue $—SiR_9R_{10}R_{11}$, $R_2$ and $R_3$, which may be identical or different, represent a hydrogen atom, a lower alkyl group having 1 to 6 carbon atoms, a carboxyl group, a ($C_1$—$C_6$) alkoxycarbonyl group or a group

—$COOSiR_9R_{10}R_{11}$; $R_4$ and $R_5$, which may be identical or different, represent a hydrogen atom, a linear or branched $C_1$—$C_{20}$ alkyl group, a formyl group, a linear or branched $C_2$—$C_{20}$ acyl group, a linear or branched $C_3$—$C_{20}$ alkenoyl group, a group $R_6OSO_2$—, a group —$SiR_9R_{10}R_{11}$, a group —$P(O)(OR_6)_2$, an aralkyl group, or alternatively $R_4$ and $R_5$, with the oxygen atoms to which they are attached, form a ring optionally containing a carbonyl group when at least one of the groups $R_1$, $R_2$ or $R_3$ is other than hydrogen, or alternatively a thiocarbonyl group, a residue $>P(O)OR_6$, a group $>CR_7R_8$ or a methylene group, $R_6$ representing a hydrogen atom or a lower alkyl group having 1 to 6 carbon atoms, $R_7$ representing a hydrogen atom or a lower alkyl group having 1 to 6 carbon atoms, $R_8$ representing a lower alkoxy group having 1 to 6 carbon atoms or a mono- or dialkylamino group and $R_9$, $R_{10}$ or $R_{11}$, which may be identical or different, representing linear or branched lower alkyl groups having 1 to 6 carbon atoms, at least one of the substituents $R_1$, $R_2$, $R_3$, $R_4$ and $R_5$ being other than hydrogen; and the corresponding salts with alkali metals, alkaline earth metals or amines.

3. Use according to Claim 1 or 2, characterized in that it employs a compound corresponding to the formula (I) in which $R_1$ represents hydrogen, $R_2$ and $R_3$, which may be identical or different, represent a hydrogen atom or a lower alkyl group having 1 to 6 carbon atoms, at least one of the groups $R_4$ or $R_5$ represents a linear or branched $C_1$—$C_{20}$ alkyl group, a linear or branched $C_2$—$C_{20}$ acyl group or a linear or branched $C_3$—$C_{20}$ alkenoyl group, the other substituent being hydrogen, or alternatively $R_4$ and $R_5$ simultaneously represent $SiR_9R_{10}R_{11}$, in which $R_9$, $R_{10}$ and $R_{11}$ have the meanings stated in Claim 1.

4. Use according to Claim 1 or 2, characterized in that the compounds are selected from 5,6-dibenzyloxyindole, 5-benzyloxy-6-methoxyindole, 6-benzyloxy-5-methoxyindole, 6-hydroxy-5-methoxyindole, 5-hydroxy-6-methoxyindole, 5,6-diacetoxyindole, 6-acetoxy-5-methoxyindole, (5 or 6)-acetoxy-(6 or 5)-hydroxyindole, 5,6-dibenzyloxy-2-carbethoxyindole, 5,6-dibenzyloxy-2-carboxyindole, 5,6-dihydroxy-2-carbethoxyindole, 5,6-dihydroxy-2-carboxyindole, 5,6-dibenzyloxy-2-methylindole, 5,6-dihydroxy-2-methylindole, 5,6-dibenzyloxy-3-methylindole, 5,6-dihydroxy-3-methylindole, (5 or 6)-formyloxy-(6 or 5)-hydroxyindole, (5 or 6)-acetoxy-(6 or 5)-formyloxyindole, 6-formyloxy-5-methoxyindole, 6-benzyloxy-5-butoxyindole, 5-butoxy-6-hydroxyindole, 5-benzyloxy-6-butoxyindole, 6-butoxy-5-hydroxyindole, (5 or 6)-hydroxy-(6 or 5)-trimethylsilyloxyindole, 5,6-bis(trimethylsilyloxy)indole, 5,6-(1-ethoxyethyl-1,1-dioxy)-indole, 5,6-dihydroxyindole cyclic phosphodiester, 5,6-thiocarbonyldioxyindole, 5-methoxy-6-trimethylsilyloxyindole, 5,6-bis(trimethylsilyloxy)-2-methylindole, 5,6-carbonyldioxy-2-methylindole, (5 or 6)-hydroxy-(6 or 5)-myristoyloxyindole, 5,6-dimyristoyloxyindole, (5 or 6)-hydroxy-(6 or 5)-oleyloxyindole, 5,6-dioleyloxyindole, 5,6-bis(trimethylsilyloxy)-2-carbethoxyindole, 5,6-bis(trimethylsilyloxy)-2-trimethylsilyloxycarbonylindole, 5,6-bis(trimethylsilyloxy)-3-methylindole, 6-hexadecyloxy-5-methoxyindole, 6-hexadecyloxy-5-hydroxyindole, 5-hexadecyloxy-6-hydroxyindole, 5,6-dipivaloyloxyindole, (5 or 6)-hydroxy-(6 or 5)-pivaloyloxyindole, 5,6-dihexanoyloxyindole, (5 or 6)-hexanoyloxy-(6 or 5)-hydroxyindole, 5,6-dibutanoyloxyindole and (5 or 6)-butanoyloxy-(6 or 5)-hydroxyindole.

5. Use according to any one of Claims 2 to 4, characterized in that the cosmetic medium of the composition consists of an anhydrous medium.

6. Use according to any one of Claims 2 to 5, characterized in that the cosmetic medium of the composition contains at least one solvent selected from lower alkanols, ethylene glycol, propylene glycol, dipropylene glycol, tripropylene glycol, ethylene glycol monomethyl, monoethyl and monobutyl ethers and ethylene glycol monoethyl ether acetate.

7. Use according to any one of Claims 2 to 5, characterized in that the cosmetic medium of the composition contains or consists exclusively of fats.

8. Use according to any one of Claims 2 to 7, characterized in that the composition is presented in the form of a lotion, a gel, an emulsion, a balm, a stick, an oil, a milk or a cream.

9. Use according to any one of Claims 1 to 8, characterized in that the composition contains, in addition, cosmetic adjuvants customarily used in topical compositions, selected from thickeners, demulcents, humectants, superfatting agents, sequestering agents, emollients, wetting agents, surfactants, polymers, preservatives, antifoams, fragrances, emulsifiers, bactericides and other ingredient customarily used in cosmetics.

10. Use according to either one of Claims 8 and 9, with a view to providing additionally for the protection or maintenance of the tan, characterized in that the composition contains, in addition, sunscreens specific for UV-B radiation and/or UVA-A radiation and compatible with the compounds of formula (I).

11. Use according to any one of Claims 2 to 10, characterized in that the compounds of formula (I) are present in the composition in proportions of between 0.01 and 15% by weight relative to the total weight of the composition.

12. Process for colouring the skin in order to impart thereto a coloration substantially similar to the pigmentation resulting from natural tanning, characterized in that at least one compound of formula (I) according to Claim 1 is applied to the skin in a suitable cosmetic vehicle.

13. Cosmetic composition intended for imparting to the skin a coloration substantially similar to the pigmentation resulting from natural tanning, characterized in that it contains, in a cosmetically acceptable medium suitable for a topical application, at least one compound of formula:

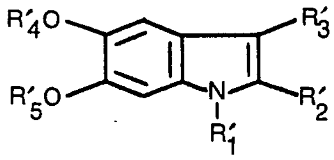

(II)

in which $R'_1$ represents a hydrogen atom or a lower alkyl group; $R'_2$ and $R'_3$, which may be identical or different, represent a hydrogen atom, a lower alkyl group, a carboxyl group, a lower alkoxycarbonyl group or a group $—COOSi(CH_3)_3$; $R'_4$ and $R'_5$, which may be identical or different, can represent a linear or branched $C_9—C_{20}$ alkyl group, a linear or branched $C_{10}—C_{20}$ acyl group, a linear or branched $C_3—C_{20}$ alkenoyl group or a group $—P(O)(OR_6)_2$, it being possible for the other group $R'_4$ or $R'_5$ to be a hydrogen atom, a $C_1—C_8$ alkyl group, a formyl group, a $C_2—C_9$ acyl group or an aralkyl group;

it being possible for $R'_4$ or $R'_5$ to denote a group $—Si(CH_3)_3$ when $R'_1$ is other than methyl; or alternatively $R'_4$ and $R'_5$, with the oxygen atoms to which they are attached, form a ring optionally containing a carbonyl group when one of the substituents $R'_1$, $R'_2$ or $R'_3$ is other than hydrogen, a thiocarbonyl group or a group $P(O)OR_6$ or $CR_7R_8$, $R_6$ denoting a hydrogen atom or a lower alkyl radical and $R_7$ representing a hydrogen atom or a lower alkyl group and $R_8$ representing a lower alkoxy group or a mono- or dialkylamino group, and the corresponding salts with alkali metals, alkaline earth metals or amines.

14. Cosmetic composition intended for imparting to the skin a coloration similar to a natural tan, characterized in that it contains at least one compound selected from 5,6-dibenzyloxyindole, 5-benzyloxy-6-methoxyindole, 6-benzyloxy-5-methoxyindole, 6-hydroxy-5-methoxyindole, 5-hydroxy-6-methoxyindole, 6-acetoxy-5-methoxyindole, (5 or 6)-acetoxy-(6 or 5)-hydroxyindole, 5,6-dibenzyloxy-2-carbethoxyindole, 5,6-dibenzyloxy-2-carboxyindole, 5,6-dihydroxy-2-carbethoxyindole, 5,6-dibenzyloxy-2-methylindole, 5,6-dibenzyloxy-3-methylindole, (5 or 6)-formyloxy-(6 or 5)-hydroxyindole, (5 or 6)-acetoxy-(6 or 5)-formyloxyindole, 6-formyloxy-5-methoxyindole, 6-benzyloxy-5-butoxyindole, 5-butoxy-6-hydroxyindole, 5-benzyloxy-6-butoxyindole, 6-butoxy-5-hydroxyindole, (5 or 6)-hydroxy-(6 or 5)-trimethylsilyloxyindole, 5,6-bis(trimethylsilyloxy)indole, 5,6-(1-ethoxyethyl-1,1-dioxy)indole, 5,6-dihydroxyindole cyclic phosphodiester, 5,6-thiocarbonyldioxyindole, 5-methoxy-6-trimethylsilyloxyindole, 5,6-bis(trimethylsilyloxy)-2-methylindole, 5,6-carbonyldioxy-2-methylindole, (5 or 6)-hydroxy-(6 or 5)-myristoyloxyindole, 5,6-dimyristoyloxyindole, (5 or 6)-hydroxy-(6 or 5)-oleyloxyindole, 5,6-dioleyloxyindole, 5,6-bis(trimethylsilyloxy)-2-carbethoxyindole, 5,6-bis(trimethylsilyloxy)-2-trimethylsilyloxycarbonylindole, 5,6-bis(trimethylsilyloxy)-3-methylindole, 6-hexadecyloxy-5-methoxyindole, 6-hexadecyloxy-5-hydroxyindole, 5-hexadecyloxy-6-hydroxyindole, 5,6-dipivaloyloxyindole, (5 or 6)-hydroxy-(6 or 5)-pivaloyloxyindole, 5,6-dihexanoyloxyindole, (5 or 6)-hexanoyloxy-(6 or 5)-hydroxyindole, 5,6-dibutanoyloxyindole and (5 or 6)-butanoyloxy-(6 or 5)-hydroxyindole.